# EUROPEAN PATENT APPLICATION

(11) **EP 4 201 361 A1**
(43) Date of publication of application: **28.06.2023**
(21) Application number: 21306947.9
(22) Date of filing: 27.12.2021
(51) Int. Cl.: A61B 34/20, A61B 34/30, A61B 90/00, A61B 17/00, A61B 90/50

(54) **REAL-TIME ELECTROMAGNETIC LOCALIZATION SYSTEM**

(71) Applicant: MinMaxMedical, 38400 Saint-Martin-d'Hères (FR)
(72) Inventor: LAVALLEE, Stéphane, 38400 SAINT MARTIN D'HERES (FR); ROUSSEAU, Sandra, 38400 SAINT MARTIN D'HERES (FR); HUGUEL, Loïc, 38400 SAINT MARTIN D'HERES (FR); CHAVE, Mickaël, 38400 SAINT MARTIN D'HERES (FR)
(74) Representative: Regimbeau

(57) **Abstract**

The invention relates to a real-time electromagnetic localization system comprising at least two locating units (10a, 10b, 10c, 10d) and a processing unit (5), each locating unit (10a, 10b, 10c, 10d) comprising at least two locating transducers (A, AA ; B, BB, C, CC; D, AA), the processing unit (5) being adapted to:
- determine a pose of a first locating transducer (A) of a first locating unit (10a) with respect to a second locating transducer (AA) of the first locating unit (10a),
- determine a pose of a first locating transducer (B) of a second locating unit (10b) with respect to a second locating transducer (BB) of the second locating unit (10b),
- determine a pose of the first locating transducer (A) of the first locating unit (10a) with respect to the first locating transducer (B) of the second locating unit (10b) based on a mechanical link (L) formed between the first locating transducer (A) and the first locating transducer (B),
- determine a pose of the second locating transducer (AA) with respect to the second locating transducer (BB) based on
- the determined pose of the first locating transducer (A) with respect to the second locating transducer (AA);
- the determined pose of the first locating transducer (B) with respect to the second locating transducer (BB);
- the determined pose of the first locating transducer (A) with respect to the first locating transducer (B).

## Description

### FIELD OF THE INVENTION

The invention concerns the field of electromagnetic localization and concerns a real-time electromagnetic localization system and a method for determining a pose of a locating transducer which forms part of a locating unit of said real-time electromagnetic localization system, in particular for determining the pose of a surgical instrument to which said transducer is attached. For instance, the system of the invention can be used to navigate a surgical instrument during a surgical procedure.

### BACKGROUND OF THE INVENTION

Computer-assisted surgery, surgical navigation, and surgical robotics all rely on the measurement of the relative position and orientation between several trackers attached rigidly to anatomical structures or bones, to surgical instruments, to robot ends, or to various sensors.

Optical locator applications are well known and widely used, though limited by the bulkiness of their large trackers and line of sight major constraints. Electromagnetic systems are particularly adapted to be used during minimally invasive surgeries, as they allow an electromagnetic transducer to be inserted into the patient through an incision, and as they are not limited by any line of sight issue.

In the present application, the word "transducer" refers to a device adapted to either emit at least one electromagnetic field or receive such an electromagnetic field.

An electromagnetic localization device generally consists of a transmitting device, also called transmitter or field generator, consisting of one or more transmitting coils rigidly linked to each other, and of a receiving device, called receiver, consisting of one or more receiving coils rigidly linked to each other. As well known in the art, the joint analysis and modeling of the magnetic fields emitted by the transmitting coils and the fields measured by the receiving coils makes it possible to determine the position and / or the orientation of the receiving device with respect to the transmitting device. Consequently, the respective position and/or orientation of objects linked to each of the receiving device and of the transmitting device can be determined as long as the geometry of the links between said transmitting or receiving devices and the objects to be tracked are known.

Typically, a transmitter comprises three coils and a receiver comprises three coils. For instance, the transmitter can comprise larger coils than the receiver. Therefore, nine measurements are obtained, and the six independent degrees of freedom of the position and orientation of a transform matrix between the receiver reference system and the transmitter reference system can be determined, for instance by least squares optimization methods. In many systems, it is possible to use one transmitter and several receivers, in order to determine the relative positions and orientations of these receivers with respect to each other. For example, one receiver can be attached to a bone, and another one to the tip of an instrument, so that the relative pose of the bone with respect to the tip of the instrument can be determined.

Electromagnetic localization devices are well known in the literature, as for example in the document "Magnetic Position and Orientation Tracking System" by Frederick H. Raab et al. published in IEEE Transactions on Aerospace and Electronic Systems VOL. AES-15, No. 5 September 1979, or the document" La localisation spatiale d'outils chirurgicaux par systemes électromagnétiques alternatifs. Applications et domaines de validite des modérations numeriques" by Joffrey Paille - Thesis, Universite Joseph-Fourier - Grenoble I, 2004. HAL Id : tel-00006125, version 1.

However, magnetic localization devices, also referred to as electromagnetic localization devices, may be prone to disturbances of the magnetic field between the transmitter and the receiver:
- Electrically conductive materials can give rise to eddy currents when these materials are placed in a varying magnetic field. The eddy currents that then circulate in this disturbing material in turn generate a disruptive magnetic field,
- Magnetic materials (for example: ferromagnetic, diamagnetic, paramagnetic, antiferromagnetic, ferrimagnetic) can distort magnetic field they are placed in. This distorted field leads to incorrect measurement.

In a typical operating room, there are many disturbing materials. Most of the surgical instruments for instance are made of such disturbing materials. In typical applications of surgical robotics and surgical navigation, the average distance between the surgical instrument and the anatomical structure to be treated can be up to 30 cm or even 50 cm. Furthermore, to achieve the level of precision expected for a surgical procedure, the distance between each of the transmitter and the receiver and the closest disturbing material should be at least 4 or even 5 times the working distance of the electromagnetic localization device which corresponds to a distance measured between the transmitter and the receiver of a same electromagnetic localization device. This means, for example, that the floor and ceiling of a room of standard height can be problematic for measuring a working distance of 30 cm or more as the distance between the operating table and the floor or the ceiling is, in a typical operating room, inferior to about 1 or 2 meter(s). Even if the working distance of the electromagnetic localization device is reduced to 15 cm, the closest disturbing material must be at a distance of at least 75 cm from the transmitter and from the receiver of such electromagnetic localization device, which is difficult to warranty.

Currently used electromagnetic localization devices are thus not adapted to be used in surgical robotics and surgical navigation. There have been many attempts to overcome these issues.

Document US7957925 describes a method of compensation of measurement artifacts generated by disturbing materials.

Document US20170202627 describes an electromagnetic system for tracking a surgical tool comprising a plurality of subsets of field generator coils disposed along edge portions of a surgical bed.

However, the solutions of the prior art are not satisfactory, since they offer only partial solutions and many applications still cannot use the existing electromagnetic localization systems which lack reliability due to their sensitivity to the presence of disturbing materials.

The present invention proposes to design an electromagnetic localization system formed as a chain of several electromagnetic localization devices, each comprising one transmitter and one receiver, and wherein two adjacent electromagnetic localization devices are linked in a known manner. Advantageously, the receiver and the transmitter of a same electromagnetic localization device can thus be sufficiently close to each other to ensure that no disturbing material will interfere with the measurement of the concerned magnetic field.

### SUMMARY OF THE INVENTION

Therefore, the invention is aimed at the aforementioned drawbacks by providing a real-time electromagnetic localization system comprising at least two locating units and a processing unit, each locating unit comprising at least two locating transducers wherein at least one locating transducer is a transmitter adapted to emit at least one magnetic field and at least one other locating transducer is a receiver adapted to receive and measure the magnetic field emitted by the transmitter, the processing unit being adapted to:
- determine a pose of a first locating transducer of a first locating unit with respect to a second locating transducer of the first locating unit based on the measurement of the magnetic field emitted by the transmitter of said first locating unit,
- determine a pose of a first locating transducer of a second locating unit with respect to a second locating transducer of the second locating unit based on the measurement of the magnetic field emitted by the transmitter of said second locating unit,

- determine a pose of the first locating transducer of the first locating unit with respect to the first locating transducer of the second locating unit based on a mechanical link formed between the first locating transducer of the first locating unit and the first locating transducer of the second locating unit, said mechanical link being formed such that its geometry is known or can be determined,
- determine a pose of the second locating transducer of the first locating unit with respect to the second locating transducer of the second locating unit based on
   - the determined pose of the first locating transducer of the first locating unit with respect to the second locating transducer of the first locating unit;
   - the determined pose of the first locating transducer of the second locating unit with respect to the second locating transducer of the second locating unit;
   - the determined pose of the first locating transducer of the first locating unit with respect to the first locating transducer of the second locating unit.

In the present document, the words "mechanical link" indistinctly refer to a purely mechanical link or to an electro-mechanical link.

The invention may be complemented by one or several of the following features, alone or in combination.

The mechanical link is a rigid and straight bar.

The mechanical link is a rigid and curved bar. For instance, such a shape permits to link transducers arranged on either side of an anatomical structure.

The mechanical link is mobile in translation and/or rotation and the processing unit is adapted to calculate a transformation determining the translation and/or rotation of the first locating transducer of the first locating unit with respect to the first locating transducer of the second locating unit.

The mechanical link is formed as an articulated device which comprises at least one joint and at least one encoder on each joint. The at least one encoder is thus adapted to send an information to the processing unit concerning the respective pose of the articulated device one either side of the concerned joint, so that said processing unit can determine the geometry of the mechanical link at any time.

The mechanical link is formed as an articulated device which comprises at least one joint driven by at least one motor controlled by the processing unit.

The mechanical link is adapted to be mounted on an articulated device which is adapted to be locked in a fixed position.

The mechanical link is adapted to be mounted on a robot which is configured to adjust the position of the first locating transducer of the first locating unit and the first locating transducer of the second locating unit such that the first locating transducer of the first locating unit is in an optimal range with respect to the second locating transducer of the first locating unit and such that the first locating transducer of the second locating unit is in an optimal range with respect to the second locating transducer of the second locating unit and such that an indicator of measurement quality of the emitted magnetic fields is optimized. By "optimal range", we here mean that a distance measured between the concerned transducers is the smallest possible, in order to avoid the effects of disturbing materials which can be in the surroundings.

The mechanical link is adapted to be non-invasively attached to a patient, and/or to a patient's support device and/or to a chest protector worn by a user of the real-time electromagnetic system.

The mechanical link comprises one or more electrically non-conductive material(s), non-magnetic material(s), and/or material(s) with low thermal expansion coefficient.

According to an embodiment of the invention, the real-time electromagnetic localization system can comprise at least a third locating unit comprising at least two locating transducers wherein at least one locating transducer is a transmitter adapted to emit at least one magnetic field and at least one other locating transducer is a receiver adapted to receive and measure the magnetic field emitted by the transmitter, a first locating transducer of the third locating unit being mechanically linked to the first locating transducer of the first locating unit and a second locating transducer of the third locating unit being mechanically linked to the first locating transceiver of the second locating unit, said mechanical links being formed such that their respective geometries are known or can be determined, the processing unit being adapted to:
- determine a pose of the first locating transducers of each locating unit with respect to the second locating transducers of each concerned locating unit based on the measurement of the magnetic field emitted by the transmitter of said each locating unit;
- determine a pose of the first locating transducer of the first locating unit with respect to the first locating transducer of the third locating unit based on the mechanical link formed between said transducers,
- determine a pose of the first locating transducer of the second locating unit with respect to the second locating transducer of the third locating unit based on the mechanical link formed between said transducers,
- determine a pose of the second locating transducer of the first locating unit with respect to the second locating transducer of the second locating unit based on
   - the determined pose of the first locating transducers with respect to the second locating transducers for each locating unit,
   - the determined pose of the first locating transducer of the first locating unit with respect to the first locating transducer of the third locating unit,
   - the determined pose of the first locating transducer of the second locating unit with respect to the second locating transducer of the third locating unit.

Optionally, the real-time electromagnetic localization system of the invention can comprise at least a fourth locating unit comprising at least two locating transducers wherein at least one locating transducer is a transmitter adapted to emit at least one magnetic field and at least one other locating transducer is a receiver adapted to receive and measure the magnetic field emitted by the transmitter, a first locating transducer of the fourth locating unit being mechanically linked to the second locating transducer of the third locating unit such that the geometry of said mechanical link is known or can be determined by the processing unit, and a second locating transducer of the fourth locating unit being shared with the first locating unit, the processing unit being adapted to :
- determine a pose of the first locating transducers of each of the second, third and fourth locating units with respect to the second locating transducers of the concerned locating units,
- determine a pose of the first locating transducer of the fourth locating unit with respect to the second locating transducer of the third locating unit based on the mechanical link formed between said transducers,
- determine a pose of the second locating transducer of the fourth locating unit with respect to the second locating transducer of the second locating unit based on:
   - the determined pose of the first locating transducers of the second, third and fourth locating units with respect to the second locating transducers of the concerned locating units,
   - the determined pose of the first locating transducer of the fourth locating unit with respect to the second locating transducer,
- compare the determined poses of the second locating transducer of the first locating unit with respect to the second locating transducer of the second locating unit and determine if the difference between said poses exceeds a predefined threshold.

Advantageously, such a comparison ensures that the determined poses are correct and, consequently, to detect any disturbance of the magnetic fields which would lead to an erroneous determination of said poses.

According to an aspect of the invention, a working distance between the two locating transducers of each locating unit is at least three times smaller than a distance, the smallest, measured between one of the locating transducers of the concerned locating unit and the closest disturbing material. For instance, this working distance can be set to be less than 60 mm. Advantageously, this working distance is less than 50 mm. Another object of the present invention concerns a method for determining a pose of a locating transducer implemented by a real-time electromagnetic localization system comprising:
- at least two locating units and at least one processing unit, each locating unit comprising at least two locating transducers, wherein
   - at least one locating transducer of each locating unit is a transmitter emitting at least one magnetic field, and
   - at least one other locating transducer of each locating unit is a receiver receiving and measuring the magnetic field emitted by the transmitter,
   - a first locating transducer of a first locating unit being mechanically linked to a first locating transducer of a second locating unit, such that a pose of the first locating transducer of the first locating unit with respect to the first locating transducer of the second locating unit is known or can be determined; and
   - a processing unit;
said method comprising determining, by the processing unit:
- a pose of a first locating transducer of a first locating unit with respect to a second locating transducer of the first locating unit, based on the measurement of the magnetic field emitted by the transmitter of said first locating unit,
- a pose of a first locating transducer of a second locating unit with respect to a second locating transducer of the second locating unit based on the measurement of the magnetic field emitted by the transmitter of said second locating unit,
- a pose of the first locating transducer of the first locating unit with respect to the first locating transducer of the second locating unit based on the mechanical link formed between the first locating transducer of the first locating unit and the first locating transducer of the second locating unit,
- a pose of the second locating transducer of the first locating unit with respect to the second locating transducer of the second locating unit based on:
   ∘ the determined pose of the first locating transducer of the first locating unit with respect to the second locating transducer of the first locating unit,
   ∘ the determined pose of the first locating transducer of the second locating unit with respect to the second locating transducer of the second locating unit, and on
   ∘ the determined pose of the first locating transducer of the first locating unit with respect to the first locating transducer of the second locating unit.

The method for determining the pose of a locating transducer can be complemented by the following feature.

The first locating transducer of the first locating unit is mechanically linked to the first locating transducer of the second locating unit; wherein said method comprises an initial step of self-calibrating for determining an initial pose of the first locating transducer of the first locating unit with respect to the first locating transducer of the second locating unit by using a calibration transducer disposed proximately to the mechanical link between said first locating transducers, wherein
if one of the first locating transducers is a transmitter, the calibration transducer receives and measures a magnetic field emitted by the transmitter;
if one of the first locating transducer is a receiver, said receiver receives and measures a magnetic field transmitted by the calibration transducer; and wherein the processing unit determines from these measurements a pose of the first locating transducer of the first locating unit with respect to the first locating transducer of the second locating unit to determine a geometry of the mechanical link between said first and locating transducers.

By "proximately", we here mean that the calibration transducer is arranged close enough to the linked-transducers to be able to receive the magnetic field emitted by such linked-transducers or to permit the linked-transducers to receive and measure the magnetic field emitted by the calibration transducer.

The present invention finally concerns a computer program product comprising program code instructions for the execution of the steps of the method as described above when said program is executed by a processing unit of a real-time electromagnetic localization system as described above.

### BRIEF DESCRIPTION OF THE DRAWINGS

Other features and advantages of the invention will appear in the following description. Embodiments of the invention will be described with reference to the drawings, in which
- Figure 1 illustrates, schematically, a real-time electromagnetic localization system according to the invention in a surgical procedure;
- Figure 2 illustrates, schematically, the real-time electromagnetic localization system according to a first embodiment of the invention;
- Figure 3 illustrates, schematically, the real-time electromagnetic localization system according to a first variant of the first embodiment of the invention;
- Figure 4 illustrates, schematically, the real-time electromagnetic localization system according to a second variant of the first embodiment of the invention;
- Figure 5 illustrates, schematically, the real-time electromagnetic localization system according to the first embodiment illustrated on figure 2 in a calibration configuration;
- Figure 6 illustrates, schematically, the real-time electromagnetic localization system according to a second embodiment of the invention;
- Figure 7 illustrates a method for determining a pose of a locating transducer according to the invention;
- Figures 8a and 8b illustrate, schematically, the real-time electromagnetic localization system according to two alternatives, in a spine application;
- Figures 9a and 9b illustrate, schematically, two embodiments of the real-time electromagnetic localization system in a knee application;
- Figure 10 illustrates, schematically, the electromagnetic localization system used during a tibial osteotomy;
- Figure 11 illustrates, schematically, the real-time electromagnetic localization system according to a third variant of the first embodiment, the real-time electromagnetic localization system being illustrated as used for an intervention on a tibial bone;
- Figure 12 illustrates, schematically, the electromagnetic localization system in an ankle application;
- Figure 13 illustrates, schematically, the real-time electromagnetic localization system used concurrently with an augmented reality device.

### DETAILED DESCRIPTION OF THE INVENTION

In the following specification, the word "pose" is defined as the position and/or orientation of an object with respect to another object. It can for instance be represented as a transform matrix between a reference system attached to a first object and a reference system attached to the other object.

As previously mentioned, the words "locating transducer" refer to a device adapted to either emit at least one electromagnetic field or receive such an electromagnetic field. Also, the words "locating transducer" and "transducer" are used without any distinction.

### Real-time electromagnetic localization system

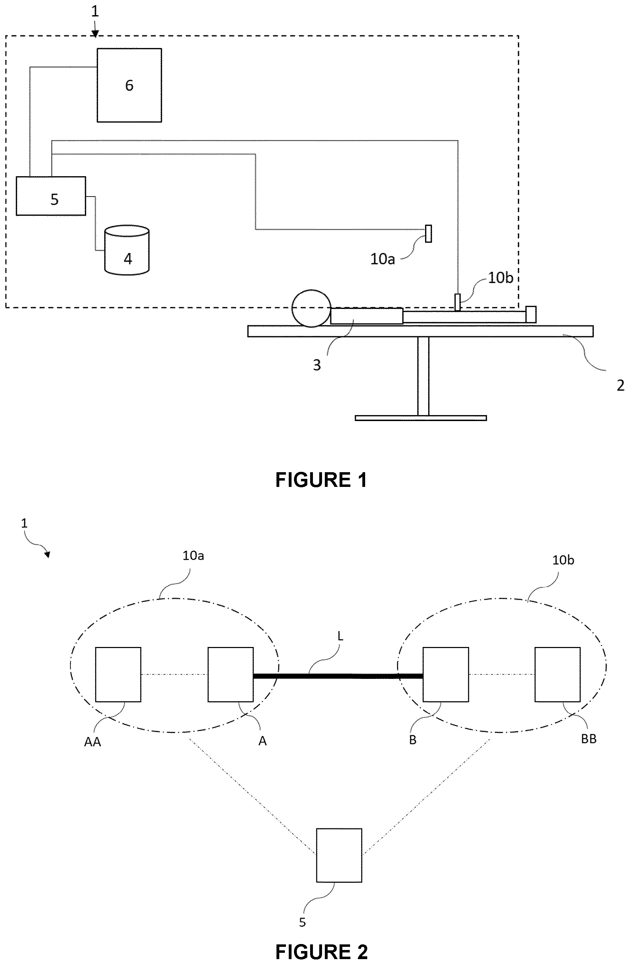

Figure 1 illustrates a real-time electromagnetic localization system 1 used in a medical environment comprising an operating table 2 on which a patient 3 may be disposed. In particular, the patient 3 may lie on this table 2 for a surgical leg procedure for instance. The real-time electromagnetic localization system 1 may also be used in various types of medical interventions, including surgery and interventional radiology.

The real-time electromagnetic localization system 1 comprises a storage unit 4 configured for storing at least signals and/or data, and at least one processing unit 5 for processing those signals and/or data images in particular in a method to track the movement of various objects in the surgical room, to facilitate the navigation of a medical instrument (see hereafter). Furthermore, the real-time electromagnetic localization system 1 can be associated with one or several display(s) 6 for the display of images or other parameters that the practitioner may need, for purposes of displaying the relative positions and orientations of the tracked objects to the practitioner.

By display we mean any suitable device for displaying information to the practitioner: means suitable for augmented reality, computer screen, means for projecting images, etc. In any case, the invention cannot be limited to a particular display.

The real-time electromagnetic localization system 1 permits, in particular, to facilitate the gesture of the practitioner or control the movement of a robot when he/it manipulates an instrument inserted into a patient by locating the instrument during the navigation, relative to the patient. The real-time electromagnetic localization system 1 may use one or several locating transducer(s) fixed to a patient fiducial for tracking purposes.

Advantageously, the real-time electromagnetic localization system of the invention, allows an iterative functioning, thus permitting a real-time localization. For instance, the frame rate can be between 10 Hz and 1000 Hz.

The instrument is, preferably, a linear instrument such as a trocar, a needle, a drill, a burr, a screw driver or equivalent. Alternatively, the instrument may be a planar instrument with a principal axis, such as a saw with the median axis of the cutting plane.

### Locating unit

To locate one object relative to another object in a reference system associated with one of the objects, the real-time electromagnetic localization system 1 comprises at least two locating units 10, wherein each locating unit may comprise at least two locating transducers: at least one magnetic field transmitter and at least one magnetic field receiver. The magnetic field receiver is configured to receive and measure the magnetic field emitted by the magnetic field transmitter for the purpose of determining the orientation and/or position of this magnetic field transmitter with respect to the magnetic field receiver. Subsequently, the term "locating transducer" represents either a magnetic field receiver or a magnetic field transmitter. The magnetic field transmitter(s) and the magnetic field receiver(s) are each associated with an object. Also, the words "locating transducer" and "transducer" are used without any distinction. In the description hereafter, a magnetic field receiver is also called a magnetic receiver or receiver, and a magnetic field transmitter is also called a magnetic transmitter or transmitter.

Hereafter, the words "working distance of a locating unit" designates a distance measured between the transmitter and the receiver of one same locating unit. "Location of an object" or "localization of an object" means that it is possible to determine the pose of the concerned object, i.e. the position of the object for example carrying a magnetic transmitter, as well as its orientation or its inclination with respect to another object, from magnetic data transmitted by said magnetic field transmitter and measured by a magnetic field receiver carried by said other object. In the present case, as will be seen later, said object can be fixed or mobile. Moreover, in the present description, the use of the word "magnetic" can be replaced by the word "electromagnetic" in the sense that the source - the magnetic transmitter - can be electromagnetic technology, and the magnetic receiver can also be electromagnetic technology.

A transmitter may comprise at least one transmitting coil, and a receiver may comprise at least one receiver coil. Generally, the transmitter comprises a number Ne (Ne≥1) of transmitter coils rigidly connected to each other and oriented along distinct axes. In the following specification, the words "transmitting coil", "emission coil" and "transmitter coil" are used without any distinction, as well as the words "receiver coil", "reception coil" and "receiving coil". We consider here, by way of example, the case of a system in which the transmitter consists of three emission coils oriented along three distinct axes, for example along the three axes of an orthogonal reference frame. These three axes of the respective magnetic moments of the coils can be confused with the axis of the winding of the coils in the case of a winding orthogonal to the winding axis. They may, however, be different if the winding is not 90 °, for example 45 °. When a voltage is imposed on the terminals of a transmitting coil, an electric current flows in the transmitting coil which then generates a magnetic field proportional to the current flowing through it and the shape of which depends on the characteristics of the coil (orientation, magnetic moment, shape ....).A voltage source may, for example, be used to impose a voltage across the transmitting coil resulting in the creation of a current. This voltage may, for example be sinusoidal. The Ne transmitting coils may be subjected to different frequency voltages.

Each transmitting coil can also be subjected, simultaneously or not, to at least two different frequencies voltages. In other words, the transmitter comprises Ne transmitting coils adapted to work at Nf frequencies, with Nf ≥ Ne. Likewise, the receiver generally comprises a number Nr of receiving coils (rigidly connected to each other and oriented along distinct axes). Alternatively, the receiver can comprise a number Nr of magneto-resistive sensors, such as anisotropique magnetoresistance sensors, global magneto-resistive sensor, tunnel magneto-resistive sensors or SQUID (superconducting quantum interference device) sensors. As known in the art, such magneto-resistive sensors comprise a resistive element with a resistance proportional to a value of the magnetic field they receive. Obviously, the receiver can comprise any other known electromagnetic receiver within the scope of the invention.

Here, by way of example, the case of a system in which the receiver is consisting of three receiving coils oriented along three distinct axes, for example along the three axes of an orthogonal reference.

In the presence of a variable magnetic field, a voltage proportional to the variation in the flux of the magnetic field appears in the reception coil. By measuring the voltage at the terminals of the reception coil, using a voltmeter or other similar means, or by measuring the current passing through the reception coil by an ammeter or other similar means, it is possible to determine the magnetic field to which the reception coil is subjected, provided that the characteristics of the reception coil are known, which include notably the magnetic moment of the reception coil, or at least the ratio of the magnetic moment of the reception coil.

The transmitter and the receiver are connected to a processing unit, including for example a microprocessor connected on one hand to the generator of the transmission device and on the other hand to the reading device of the reception device. The processing unit is configured to process the signals transmitted and received, and thus makes it possible to determine from the latter the pose of the receiver relative to the transmitter.

The processing unit receives information concerning the characteristics of the excitation signals applied to the transmission coils as well as information concerning the characteristics of the signals passing through the reception coils (representative of the field received by the reception coil). From the intensity of the fields captured by the reception coils, the processing unit is adapted to determine the pose of the set of reception coils with respect to the set of transmission coils.

More particularly, the processing unit determines (Ne x Nr) field values respectively corresponding to the field received by the receiver coil from the transmitter coil.

From these field values, the processing unit calculates the spatial position and orientation coordinates of the set of receiver coils with respect to the transmitter coils.

As previously explained, during a phase of use of the real-time electromagnetic localization system, the processing device uses known characteristics of the transducers and the characteristics of the field received by the receiver to determine the relative poses of the transducers.

Especially, the processing unit is adapted to determine a first transform matrix between a reference system attached to the transmitter and a reference system attached to the receiver of a same locating unit.

As detailed below, one of the transducers of each locating unit is attached in a known manner to the object to be tracked, for instance an anatomical structure or the tip of a surgical instrument, and the other transducer of such locating unit is linked to a transducer of a distinct locating unit. As will be explained, such link between the transducers of two locating unit can be mechanical, or optical as long as said link is known or can be determined by the processing unit. By "mechanical", we here mean "purely mechanical" or "electro-mechanical" within the scope of the invention.

The processing unit is thus adapted to determine a second transform matrix between reference systems respectively attached to each of the linked transducers of different locating units and therefore to determine the pose of such transducers with respect to one another. As such, the real-time electromagnetic localization system of the invention offers its user to navigate thanks to a chain of transducers, thus permitting to avoid, locally, the disturbing materials and making the whole system more reliable.

According to an aspect of the invention, the electromagnetic localization system 1 is designed such that the working distance of each locating unit (measured between the two locating transducers of a same locating unit as previously explained) is at least three times smaller than a distance, the smallest, measured between one of the locating transducers of the concerned locating unit and the closest disturbing material. This ratio is determined based on the average distance between the surgical instrument and an anatomical structure of the patient to be treated and on the material of such surgical instrument (stainless steel, or titanium for instance). According to a particular embodiment, this working distance is set to be less than 60 mm, advantageously less than 50 mm.

### Mechanical link

**Figure 2** describes a first embodiment of a real-time electromagnetic localization system comprising two locating units 10a, 10b, each locating unit comprising two locating transducers A, AA, B, BB : at least one magnetic field transmitter and at least one magnetic field receiver.

Magnetic field of the emission axes of each locating unit may be configured to work at at least one different frequency from (any) magnetic field of the emission axes of other locating unit, for example the frequency deviations between magnetic field of the emission axes of two locating units may be no less than about 500Hz.

Advantageously, to prevent any confusion between the two locating units the emission axes of each locating unit can be adapted to work at different frequencies from the frequencies at which the emission axes of the other locating unit are adapted to work.

Alternatively, the magnetic fields of two axes of two different locating units can be configured to work at the same frequency, each locating unit then being configured to work at a different time period from the other locating unit. Thus, the receiver(s) of each locating unit is adapted to measure only the magnetic field emitted by the transmitter of the same locating unit.

In any case, at least one magnetic field is associated to each emission axis. Optionally, two magnetic fields presenting two different frequencies can be associated with one same emission axis.

According to another aspect of the invention, each coil of at least one of the transmitters can be adapted to work at the same frequency than the other coils of said transmitter, each coil then being subjected to such frequency at a different time period from the other coils. Such other aspect of the invention is known in the art as "time multiplexing".

According to a particular embodiment of the invention, the two locating units can comprise a shared receiver. The first locating unit thus comprises a first transmitter, a first receiver and a second receiver and the second locating unit comprises a second transmitter, the second receiver and a third receiver. The first transmitter and the second transmitter here are both adapted to emit a magnetic field adapted to be received and measured by the second receiver. According to this particular embodiment, the first transmitter is thus adapted to work with the first and second receivers during a first period of time while the second transmitter is adapted to work with the second and the third receivers during a second period of time distinct from the first period of time.

In each locating unit 10, the transmitter and the receiver are positioned relative to each other so as to define a subspace wherein the artifacts generated by the disturbing materials are negligible. In the present document, such subspace is also referred to as the "workspace" of the concerned locating unit.

As previously mentioned, in operating conditions, the distance between the first and second locating transducers of each locating unit is at least three times smaller than a distance, the smallest, measured between one the transducers of the concerned locating unit and the closest disturbing material. Advantageously, this working distance is particularly adapted to achieve the level of precision expected for a surgical procedure. Indeed, this distance allows neglecting the influence of potentially disturbing materials (for example, the floor and ceiling of a surgical room of standard height or even metallic implant(s) already implanted in the patient's anatomical structure to be treated).

As previously mentioned, the two locating units 10 may be linked by a mechanical link. By "mechanical link", we here refer to a purely mechanical link or to an electro-mechanical link.

Preferably, the mechanical link comprises one or more electrically non-conductive material(s), non-magnetic material(s), and/or material(s) with low thermal expansion coefficient.

For example, the mechanical link can comprise plastic (such as Polyether ether ketone: PEEK, Polyoxymethylene: POM), fiberglass, ceramic, minerals (for example: marble, quartz), glass-ceramic, or carbon fiber.

As represented, a first locating transducer A (a transmitter or a receiver) of a first locating unit 10a is linked to a first locating transducer B (a transmitter or a receiver) of the second locating unit 10b. Hereafter in the description, the term "linked transducers" is used to refer to the linked transducers of different locating units, and the term "not-linked transducer" is used to refer to the transducers which are not linked to a transducer of a different locating unit.

### Rigid mechanical link

In a first configuration, the mechanical link L is rigidly fixed to the linked transducers, for example in the form of a connecting bar, of which the geometry is previously known. According to different embodiments of the invention, the connecting bar can for instance be shaped as a rigid straight bar, as an elongated bar, as a curved bar, or as a U-shaped bar. More generally, the bar can be of any shape as long as it is rigid. The practitioner can thus choose the most convenient bar, depending, for example, on the kind of surgery to be realized and on the relative poses of the tracked objects.

For instance, a straight bar has the advantage of being compact. Such a rigid mechanical link advantageously aims at creating a fixed and stable relationship between the first locating transducer of the first locating unit and the first locating transducer of the second locating unit.

In the case of an elongated straight bar, the length of such bar can be significant, for example 100 mm, 200 mm or 300 mm, provided that it remains rigid, with no deformation exceeding the required accuracy, for example 0.1 mm and 0.05 degrees.

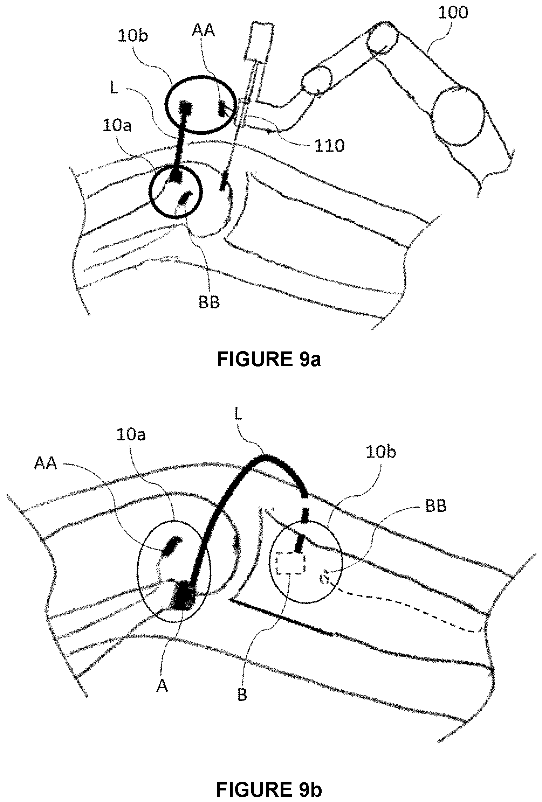

Using a curved bar has the advantage that the curved bar can have a shape adapted to the anatomical structure to be treated or the environment. For example, as shown on **figure 9b****,** a curved bar can be used to link transducers respectively arranged on an external side of a tibia and on an internal side of a femur of a same leg.

Regardless its shape or geometry, the bar forming the mechanical link can be carried by different devices. For instance, the bar can be held by a passive articulated arm adapted to be locked in position using brakes and to be adjusted manually, the base of said articulated arm being fixed to a table rail for instance or to any device in the surgical field, such as operating room lights, imaging device, surgical robot....

Alternatively, the bar can be caried by a light robot, slow, safe and powerful enough to hold said bar. Said robot is advantageously adapted to position the linked transducers in an optimal range with respect to the associated not-linked transducers. The optimal range here refer to the working distance of the concerned locating unit as defined above. The bar can also be held by a Cobot such as Universal Robot UR3 (Odense, Denmark), that can be placed manually or placed by an optimal motorized and safe process during a surgical procedure.

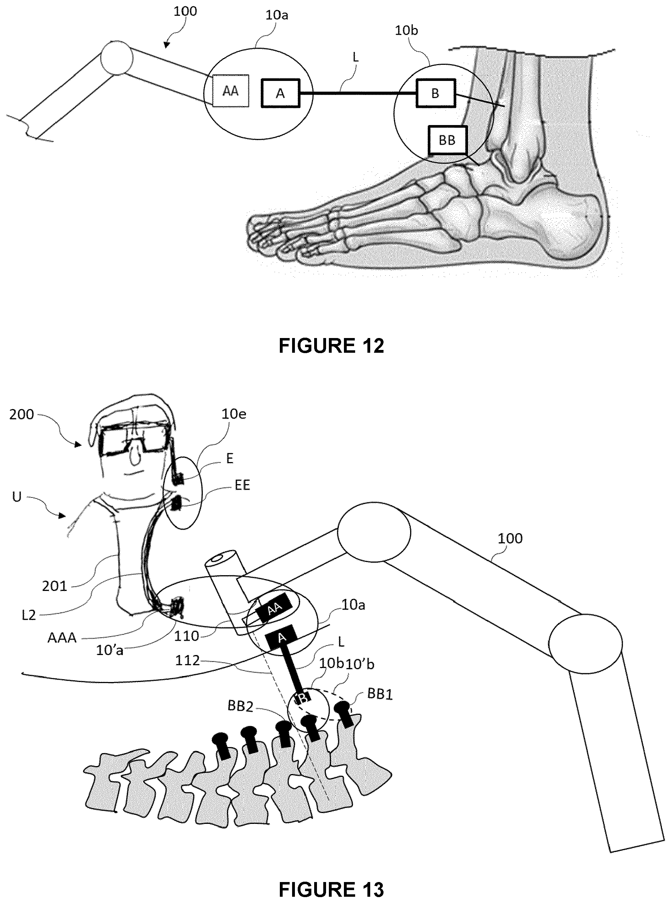

According to another alternative, the bar can also be positioned on a support fixed to the patient thanks to straps or tape, on a patient's support such as a leg holder or a cushion or on a chest protector worn by a user of the system as detailed with respect to **figure 13****.**

As seen before, by conventional means, in each locating unit 10, the magnetic field receiver is configured to measure the magnetic field emitted by the magnetic field transmitter.

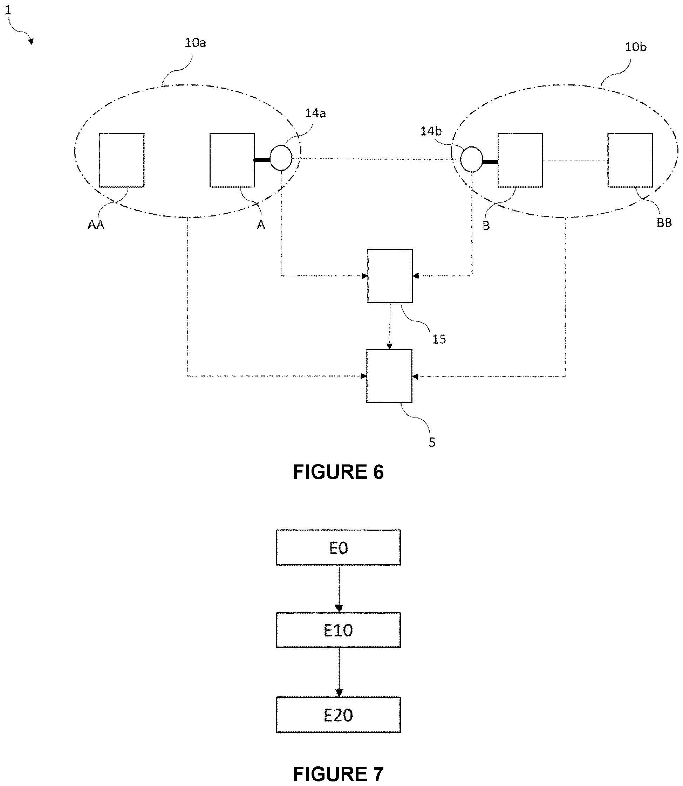

In reference to **figure 7**, in a step E10, the processing unit 5 is configured to determine from those measurements the pose, in each locating unit 10, of the not-linked transducer A, B with respect to the linked transducer AA, BB.

Knowing the geometry of the mechanical link L, and thus the pose of the linked transducers A, B of each locating unit 10a, 10b, the processing unit 5 is also configured, in a step E20, to determine a pose of the not-linked transducer AA of the first locating unit 10a with respect to the not-linked transducer BB of locating unit 10b.

Especially, the processing unit is adapted to determine the first transform matrix for each locating unit 10a, 10b, as previously described. As the geometry of the link L is here known, the processing unit can determine a second transform matrix between the reference systems respectively attached to the linked-transducer of the first locating unit and to the linked-transducer of the second locating unit. The processing unit is then adapted to combine and compute the first transform matrices and the second transform matrix in order to determine the pose of the not-linked transducers of each locating unit with respect to one another. Consequently, the processing unit can determine the relative pose of the objects to which the not-linked transducers are attached.

Advantageously, the link L between the locating units 10 allows the location of measurement points, by example distant of more than 30 centimeters away while locally protecting against surrounding disturbing material, as the link is between subspaces wherein the artifacts generated by the disturbing materials are negligible.

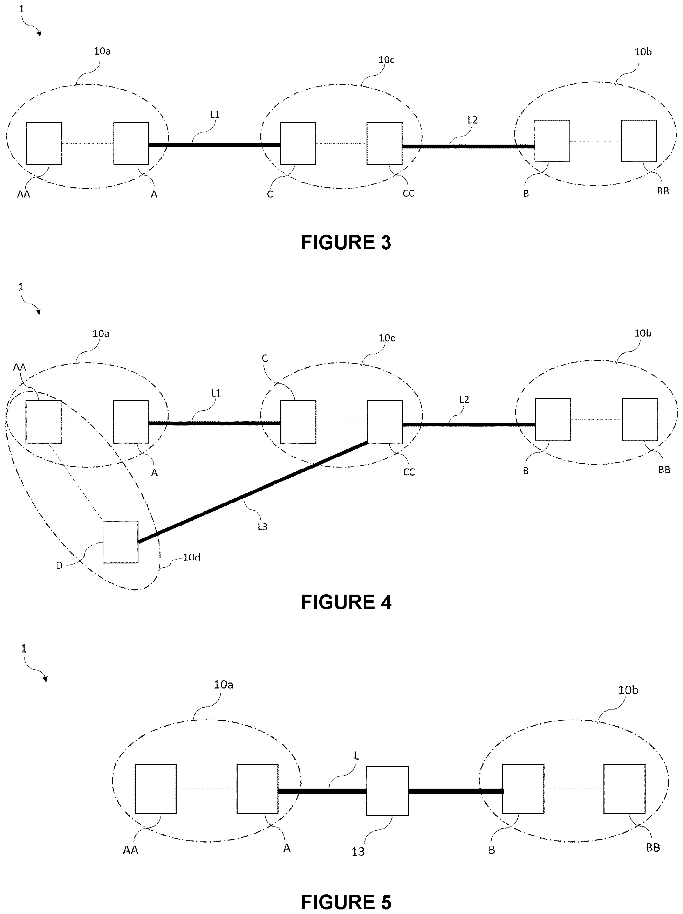

In reference with **figure 3**, it is illustrated a real-time electromagnetic localization system 1 comprising more than two locating units. Indeed, one or more intermediate locating unit(s) 10c may be located between the locating units 10a, 10b, which relative to the intermediate locating unit(s) 10c may be represented as terminal locating units 10a, 10b.

In each intermediate locating unit 10c, one locating transducer C may be connected by a mechanical link L1 to a first intermediate locating unit (not represented) or to a first terminal locating unit 10a, and the other locating transducer CC may be connected by a mechanical link L2 to a second intermediate locating unit (not represented) or to the second terminal locating unit 10b.

The functioning of the system remains similar to what have been described previously: the processing unit is adapted to determine the first transform matrices for each locating unit 10a, 10b, 10c, to determine the second transform matrices between the linked transducers A-C, CC-B and to determine the pose of the not-linked transducers AA, BB based on the combination and computing of said first and second matrices.

The links L1, L2 formed between the intermediate locating unit 10c and the terminal locating units 10a, 10b are here described as mechanical rigid links, but they can be of any other kind within the scope of the invention.

**Figure 4** illustrates yet another configuration of the real-time electromagnetic system 1 of the invention. This configuration differs from the one described with reference to **figure 3** in that it comprises a fourth locating unit 10d which comprises a first locating transducer D linked to the second locating transducer CC of the third locating unit 10c and a second locating transducer AA which is shared with the first locating unit 10a.

In this configuration, the pose of the second locating transducer AA of the first locating unit 10a with respect to the pose of the second locating transducer BB of the second locating unit 10b can be determined thanks to two distinct chains of transducers. Especially, the processing unit 5 can determine the pose of said second transducers AA, BB thanks to the first matrices associated to each of the first, second and third locating units 10a, 10b, 10c and on the second transform matrices associated with the corresponding links L1, L2 or it can determine the pose of said second transducers AA, BB, thanks to the first matrices associated to each of the second, third and fourth locating units 10b, 10c, 10d and on the second transform matrices associated with the corresponding links L2, L3.

Then, the processing unit can compare the obtained values. If a difference between these values exceeds a predefined threshold, the processing unit can be adapted to:
- alert the user that at least one of the obtained values is probably wrong, and/or
- order a displacement of at least one of the linked transducers to optimize the measurement and/or
- indicate to the user which displacement he/she needs to apply to the at least one of the linked transducers to optimize the measurement.
As previously mentioned, the processing unit can be adapted to use an indicator of quality of measurement to determine if the measurements are optimized or if the linked transducers should be displaced to optimize said measurements.

The link(s) formed between the different locating units of the system is/are thus used to manage most of the distance between the not-linked transducers, whilst the linked transducers are used to perform small distance measurements using electromagnetic localization. Using electromagnetic localization combined to the described link thus permit to navigates thanks to electromagnetic tracking while avoiding the current constraints formed by the disturbing material which are present in an operating room. In theory, the system of the invention could be used to locate transducers distant by a distance which could be up to 1 000 cm, the only limit being the stiffness of the link formed between the locating units which must be sufficient to ensure that the geometry of the link can be determined with accuracy.

### Self-calibrating mechanical link

According to an aspect of the invention, the mechanical link L between two locating units can be initially determined by a self-calibrating step E0.

**Figure 5** illustrates a configuration of the real-time electromagnetic localization system 1 to perform such self-calibrating step E0.

If the link L between the first locating transducers A and B from, respectively, the first locating unit 10a and the second locating unit 10b is between a receiver from the first locating unit 10a and a receiver from the second locating unit 10b, a calibration transducer 13 corresponding to a transmitter is used.

The receivers from the first locating unit 10a and the second locating unit 10b measure the magnetic field emitted by the calibration transducer 13.

Using these measurements, the processing unit 5 determines the pose of the receiver A to the calibration transducer 13, and determines the pose of the receiver B to the calibration transducer 13, thus allowing determining the geometry of the link L between the linked transducers A and B (both receivers) of the first locating unit 10a and the second locating unit 10b. Once that the geometry of the link L has been determined, the processing unit is adapted to determine the second transform matrix between the linked-transducers A, B, as previously described.

If the link L between the first transducers A and B from the first locating unit 10a and the second locating unit 10b is between a transmitter from the first locating unit 10a and a transmitter from the second locating unit 10b, a calibration transducer 13 corresponding to a receiver is used.

The calibration transducer 13 measures the magnetic fields emitted by the transmitters of the first locating unit 10a and the second locating unit 10b.

Using these measurements, the processing unit 5 determines the position and/or orientation of the transmitter A to the calibration transducer 13, and determines the position and/or orientation of the transmitter B to the calibration transducer 13, thus allowing determining the geometry of the link L between the linked transducers A and B (both transmitters) of the first locating unit 10a and the second locating unit 10b. Once that the geometry of the link L has been determined, the processing unit is adapted to determine the second transform matrix between the linked-transducers A, B.

If the link L between the first locating transducers A and B from the first locating unit 10a and the second locating unit 10b is between a transmitter from the first locating unit 10a and a receiver from the second locating unit 10b, said receiver is used to measure the magnetic field emitted by the transmitter from the first locating unit 10a.

Using these measurements, the processing unit 5 determines the pose of the transmitter from the first locating unit 10a relative to the receiver from the second locating unit 10b, thus allowing determining the geometry of the link L between the linked transducers A and B of the first locating unit 10a and the second locating unit 10b. Once that the geometry of the link L has been determined, the processing unit is adapted to determine the second transform matrix between the linked-transducers A, B.

These embodiments thus differ from the preceding ones in that the geometry of the mechanical link is not known in advance, but determined by the processing unit itself, thanks to the calibration transducer 13.

### Movable mechanical link

In another embodiment, unlike the first embodiment previously described, the linked transducers A and B of the first locating unit 10a and of the second locating unit 10b are mechanically linked by movable means. The link L may be movable in translation and/or rotation. If the mechanical link L is movable in translation, said link L may be in the form of continuous segments comprising articulated elements between said segments.

The articulated elements permit to position the linked transducers A and B, both in position (up to three degrees of freedom in translation) and in orientation (up to three degrees of freedom in rotation) such that the locating transducers can be positioned and oriented in space in all possible ways.

The mechanical link L may also comprise an incremental coder capable of providing information relative to the angular position of each of these segments with respect to the others.

If the mechanical link L is movable in translation, by example in the form of a slide link or a telescopic link, the mechanical link L may comprise an incremental coder capable of providing information relative to a linear transformation.

Knowing the initial geometry of the mechanical link L, and with the transformation information provided by the coder, the processing unit 5 is configured to determine the position and orientation of the linked transducers of each locating unit 10a and 10b. Consequently, the processing unit 5 is also configured to determine a position and/or an orientation of the not-linked transducer AA of the first locating unit 10a with respect to the not-linked transducer BB of the second locating unit 10b, in a similar way to what have been described with reference to the first embodiment.

According to another alternative, the mechanical link L can be formed as an articulated device which comprises at least one joint driven by at least one motor controlled by the processing unit. As the motor are driven by the processing unit, such processing unit can determine, at any time, the geometry of such link.

As in the first embodiment, the real-time electromagnetic localization system 1 can comprise more than two locating units. In this case, in each intermediate locating unit 10c, one locating transducer C may be connected by a movable mechanical link L1 to a first intermediate locating unit (not represented) or to a first terminal locating unit 10a, and the other locating transducer CC may be connected by a movable mechanical link L2 to a second intermediate locating unit (not represented) or to the second terminal locating unit 10b.

The real-time electromagnetic localization system 1 may also comprise a combination of at least one rigid mechanical link and at least one movable mechanical link without departing from the scope of the invention.

### Optical link

**Figure 6** illustrates yet another embodiment of a real-time electromagnetic localization system 1 comprising two locating units 10a, 10b, each locating unit comprising two locating transducers A, AA, B, BB: at least one magnetic field transmitter and at least one magnetic field receiver.

In each locating unit 10, the transmitter and the receiver are positioned relative to each other so as to define a subspace wherein the artifacts generated by the disturbing materials are negligible.

According to this third embodiment, in operating conditions, the distance between the first and second transducers of each locating unit is at least three times smaller than a distance, the smallest, measured between one the transducers of the concerned locating unit and the closest disturbing material.

This third embodiment differs from the first and second embodiments in that the link formed between the locating units is an optical link. A first locating transducer A (a transmitter or a receiver) of the first locating unit 10a is thus optically linked to a first locating transducer B (a transmitter or a receiver) of the second locating unit 10b.

The optical link comprises optical trackers 14a, 14b attached to each of the locating transducers A, B, and an optical localization device 15 configured to track the location of said optical trackers 14a, 14b.

The optical localization device 15 may comprise one or more optical sensors, such as charge-coupled devices or CCDs, which receive light signals from the optical trackers 14a, 14b. The optical trackers 14a, 14b can be reflective fiducials, light-emitting diodes or LEDs.

The optical localization device 15 is configured to determine the pose of the trackers 14a, 14b. As the optical trackers 14a, 14b are fixed to the transducers in a known manner, the processing unit 5 is adapted to determine the pose of the linked transducers A, B based on the determined pose of the trackers 14, 14b, and then to determine the second transform matrix associated with said linked transducers. In a similar way to what have been earlier described, the processing unit 5 is then adapted to determine the pose of the not-linked transducers AA, BB, based on the determined first matrices of each locating unit and on the second transform matrix between the reference systems attached to each linked transducer.

According to a variant of this third embodiment, the linked transducers can be attached to optical localization devices adapted to determine their pose with respect to a tracker arranged in a fixed position. Obviously, other arrangements can be implemented within the scope of the invention. For instance, one optical tracker can be fixed to one of the linked transducers and the optical localization device can be fixed to the other of the linked transducers.

### Spine application

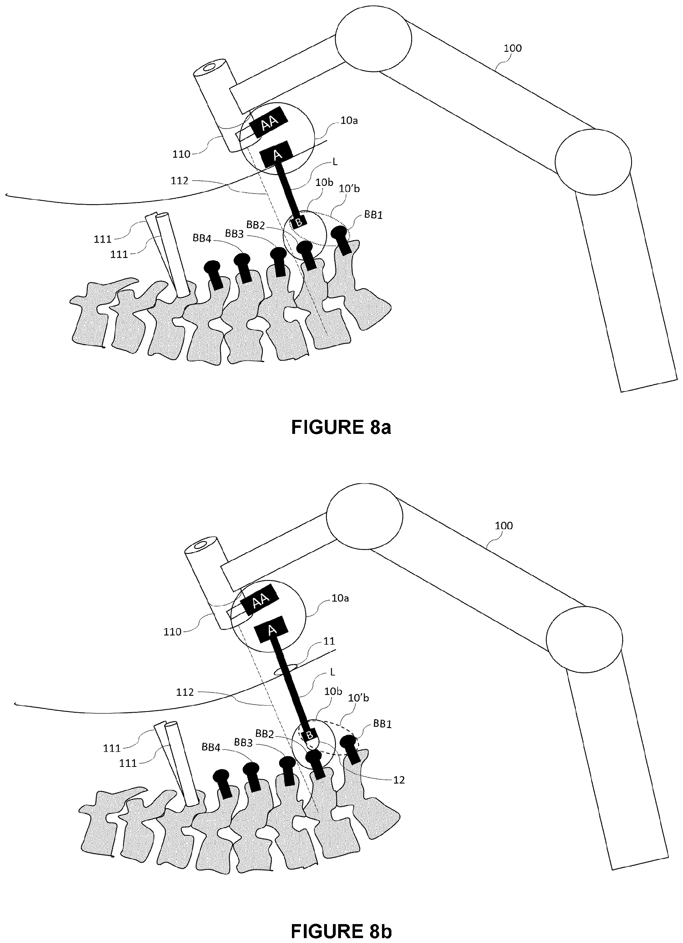

As illustrated on **figure 8a**, the real-time electromagnetic localization system 1 of the invention can be used to navigate a surgical tool 112 during spine surgeries. The surgical tool 112 is here represented schematically with dotted lines.

According to the example shown, the linked transducers A, B are both transmitters and the not-linked transducers AA, BB1, BB2..BBn are receivers. As illustrated, the first locating unit 10a comprises the receiver AA attached to an end-effector 110 of a robotic arm 100 and the transmitter A positioned on the skin of the patient to be treated. The second locating unit 10b comprises the transmitter B linked to the transmitter A of the first locating unit 10a, here thanks to a mechanical rigid link L. Obviously, this link could take any other shape within the scope of the invention.

According to the illustrated example, the second locating unit 10b comprises several receivers, each attached to one vertebra. The processing unit 5 is thus adapted to know, in real time, the pose of the end-effector 110 of the robotic arm 100 with respect to each of said vertebrae. Especially, the receivers are attached to the vertebras which are within the working distance from the transmitter B. As illustrated, the second locating unit 10b, here is formed by the linked transducer B and by one of the receivers BB attached to the vertebrae. As such, several "second locating units" are formed, two of them being referenced 10b and 10'b on figure 8a. As previously explained, the real-time electromagnetic localization system of the invention can be adapted to work, sequentially, at different frequencies, thus allowing the processing unit to discriminate each of the receivers.

Advantageously, the real-time electromagnetic system of the invention, allows an iterative functioning, thus permitting a real-time localization. For instance, the frame rate can be between 10 Hz and 1000 Hz.

**Figure 8a** also illustrates, schematically, implants 111 which have already been implanted in one of the patient's vertebrae, for instance during a previous surgery. The electromagnetic localization system of the invention advantageously permits to avoid errors in the magnetic field measurements which would otherwise occur in the vicinity of such implants. Such errors are avoided, thanks to the position of the transducers of each formed locating unit which are sufficiently close to each other with respect to the distance between said transducers and said implants. As previously mentioned, the working distance of each locating unit is three times smaller than the distance measured between any of the concerned transducers and the closest disturbing material, here formed as the implants.

Optionally, the electromagnetic localization system 1 of the invention can comprise an indicator of measurement quality thanks to which the processing unit can determine if the measurements of the electromagnetic fields of each locating units seem relevant. If an error is detected, the processing unit can be adapted to set an alarm to alert the user to not use the concerned measurement. Alternatively, the processing unit can be adapted to indicate the user how to displace the linked transducers to improve the measurement, or it can also, when possible, send an order to the robot holding said linked transducers for it to displace such transducers and improve the measurements.

There are many indicators that can be computed for such purpose, such as simply the residual errors resulting from overdetermined systems using least squares methods (more measurements than unknowns). As mentioned above, the position of the link, and consequently of the linked transducers, can be adjusted manually or automatically until the quality indicator is below a given threshold. The quality indicator can be calculated for several positions of the linked transducers and the directions where it decreases for each concerned locating unit are indicated to the user or to the robot that carries the linked transducers so that they can be moved in the right direction. Typically, if a disturbing material is placed on one side of a transducer, the other locating transducer of the same locating unit will have to be placed on the other side in order to reduce or annihilate its influence.

**Figure 8b** illustrates an alternative to the embodiment illustrated on figure 8a. This alternative differs from the embodiment illustrated on figure 8a in that the mechanical link L is longer and in that such mechanical link is maintained on the skin of the patient thanks to a maintaining device 11, here formed as a ring. Advantageously, using a longer mechanical link permits to ensure that the first locating transducer B of the second locating unit 10b is always near the second locating transducer BB of the second locating unit 10b, regardless the size of the patient. Thus, using a longer mechanical link L permits to treat obese patients as well as fit patients with the same system.

Optionally, the first locating transducer B of the second locating unit 10b can be equipped with a spacer 12 adapted to ensure that there is always a minimal distance between the coils of the first and of the second locating transducers of the second locating unit 10b. For instance, the spacer 12 of the first locating transducer B can be formed such that when it is arranged in contact with the second locating transducer BBn of the concerned locating unit, the distance between the concerned coils is greater or equal to 20 mm.

### Knee applications

**Figures 9a and 9b** illustrate the real-time electromagnetic localization system 1 of the invention used during an intervention on a knee or while preparing it.

**Figures 9a and 9b** illustrate configurations wherein the linked transducers A, B of two locating units 10 are positioned on the patient's skin in a non-invasive manner.

**Figure 9a** illustrates a configuration wherein the second locating transducer AA of the first locating unit 10a is attached to the end-effector 110 of a robotic arm 100 while the second locating transducer BB of the second locating unit 10b is attached to the patient's bone, in this case a femur. The linked transducers A, B of the first and second locating units 10a, 10b are here non-invasively fixed to the skin of the patient, for instance thanks to surgical tape.

In a similar way to what have been described above, the processing unit is adapted to determine a pose of the first locating transducer A of the first locating unit 10a with respect to the second locating transducer AA of this first locating unit 10a, based on the measurement of the magnetic field emitted and received by such locating transducers A, AA. Then, the processing unit is adapted to determine the pose of the transducers B, BB of the second locating unit 10b with respect to one another based on the magnetic field transmitted and received by such locating transducers B, BB. Finally, based on the determined poses of the transducers of each locating unit and on the known - or determined - link L formed between such locating units, the processing unit is adapted to determine the pose of the second locating transducer AA of the first locating unit 10a with respect to the second locating transducer BB of the second locating unit 10b. As the locating transducers are attached, respectively, to the end-effector and to the femur, the processing unit is adapted to subsequently determine the pose of the end-effector with respect to the concerned anatomical structure - here a femur. Advantageously, the characteristics of the surgical instrument held by the end-effector can be recorded in the storage unit of the system so that the processing unit can determine the pose of the surgical instrument with respect to the anatomical structure based on the determined pose of the end-effector with respect to such anatomical structure.

**Figure 9b** illustrates a situation wherein the electromagnetic localization system is used to determine the relative poses of two bones, in this case a femur and a tibia. Advantageously, the link L formed between the transducers A, B of the two locating units 10 is a rigid and curved bar, thus allowing to position the locating units 10 on either side of the leg. According to the illustrated example, the not-linked transducers AA, BB are attached, respectively, to an internal face of the femur and to an external face of the tibia. The transducers B, BB of the second locating unit 10b are represented in dotted lines as they are hidden behind the leg.

The functioning of the system still remains identical to what have been described above: the processing unit is adapted to determine the pose of the transducers A, AA; B, BB of each locating unit 10a, 10b, then to determine the pose of the linked transducers A, B, based on the link L formed between such transducers and finally to determine the pose of the not-linked transducers - and consequently of the objects to which such not-linked transducers are attached - based on the pose of the transducers of each locating unit and on the pose of the linked-transducers.

### Osteotomy application

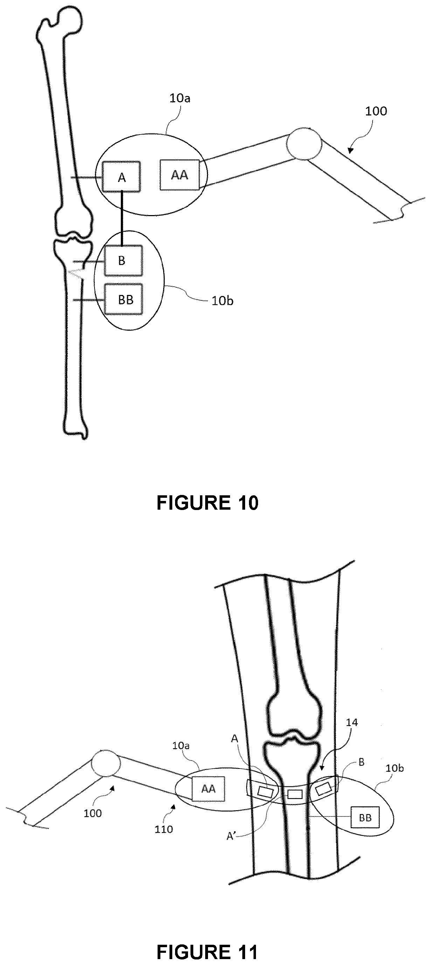

**Figure 10** illustrates, schematically, a tibial osteotomy procedure using the electromagnetic localization system of the invention. An osteotomy is a surgery which consists in removing part of a bone. For instance, an osteotomy can aim at re-aligning the leg of a patient who is suffering of arthrosis. During such a procedure, it can be useful and time saving to be able to monitor the amount of bone removed, and some alignment parameters which are supposed to be modified during the procedure. To measure those parameters in real-time, the transducers B, BB of the second locating unit 10b can for instance be both attached to the tibia, on either side of the planned osteotomy, the first locating transducer B of the second locating unit 10b being linked to the first locating transducer A of the first locating unit 10a which is attached to the femur and the second locating transducer AA of the first locating unit 10a is attached to the robotic arm 110 which holds the surgical instrument, even if not represented on figure 10. As described above, this arrangement thus permits the user to know, in real time, the amount of bones that have been removed - thanks to the determined pose of the transducers B, BB of the second locating unit 10b with respect to one another - and also the pose of the robotic arm 100, and consequently of the surgical tool hold by such robotic arm, with respect to both the femur and the tibia.

### Intervention on a tibial bone

**Figure 11** illustrates, schematically, the real-time electromagnetic localization system according to a third variant of the first embodiment of the invention, such real-time electromagnetic localization system being represented as used for an intervention on a tibial bone. Obviously, this is only an example and the electromagnetic localization system according to this third variant of the first embodiment could be used with any long bone, such as a femur, a radius etc, or it can even be used around any anatomical joint, such as a knee for instance.

Figure 11 is a schematic representation of a knee joint as the bones are made visible as well as part of the soft tissues which surround it.

During an intervention, a surgeon may need to intervene on opposite parts of a same bone, or even on several bones of a same member, such that a femur and a tibia of a lower limb for instance. To facilitate the procedure, the present invention proposes to use a locating device 14 formed as a chain of at least three linked-transducers A, A', B.

As illustrated on figure 11, such locating device 14 can be arranged around the concerned member, here a tibial bone, so that the real-time localization system can be used regardless the position of the end-effector 110 of the robotic arm 100. According to the illustrated embodiment, the locating device 14 can be formed as a rigid curved bar carrying several locating transducers A, A', B, so that their pose with respect to one another are known and can be registered in the storage unit of the system.

In a similar way to what have been previously described, the real-time localization system according to the variant illustrated on figure 11 comprises the first locating unit 10a encompassing the first locating transducer A and the second locating transducer AA, and the second locating unit 10b encompassing the first locating transducer B and the second locating transducer BB, the first locating transducers A, B of each locating unit 10a, 10b being linked to each other as they are part of the locating device 14. Thus, the illustrated variant differs from what have been previously described in that more than two locating transducers are linked to each other. According to the illustrated example, the locating device 13 comprises three first locating transducers A, A', B linked to each other, the second locating transducer AA of the first locating unit 10a being attached to the end-effector of the robotic arm 100 and the second locating transducer BB of the second locating unit 10b being attached to the tibial bone, advantageously in a region where the surgeon has to intervene. The processing unit is thus adapted to determine the pose of the locating transducers of the first locating unit 10a with respect to one another, to determine the pose of the locating transducers of the second locating unit 10b with respect to one another and to use said determined poses and the link formed between the linked-transducers A, B to determine the pose of the not-linked locating transducers AA, BB.

Advantageously, this configuration permits to always have at least one first locating transducer A, A' near the second locating transducer AA attached to the end effector. By "near", we here mean that a distance measured between said transducers is less than 60 mm, advantageously less than 50 mm. As such, the control unit can determine, at any time, the pose of the end-effector of the robotic arm with respect to the region where the surgeon has to intervene.

Obviously, figure 11 only illustrates an example of how to use the localization device according to the third variant of the first embodiment, and this localization device could be used in a wide range of situations within the scope of the invention. For instance, the locating device 14 could be used as illustrated to locate elements pertaining to the femur or it could be arranged around the femur and be used to locate elements pertaining to the femur and/or to the tibia. According to another alternative, the locating device could also be arranged around the knee and be used to locate elements pertaining to the femur and/or the tibia. Obviously, it could also be used on a superior member without departing from the scope of the invention.

Also, the shape of the locating device 14 can vary from what is illustrated on figure 11 without departing from the scope of the invention.

### Ankle application

**Figure 12** illustrates, schematically, the real-time electromagnetic system of the invention used in an ankle arthroplasty. During such a procedure, at least the pose of the tibia with respect to the talus needs to be tracked. According to the illustrated embodiment, at least the first locating transducer B of the second locating unit 10b is attached to the tibia and the second locating transducer BB of the second locating unit 10b is attached to the talus. The first locating transducer A of the first locating unit 10a is linked to the first locating transducer B of the second locating unit 10b thanks to a rigid bar. Obviously, this is only an example and the concerned transducers could be linked by any other means within the scope of the invention, as described in the present application. The second locating transducer AA of the first locating unit 10a is attached to the end effector of a robotic arm 100 adapted to hold the needed surgical tools. In a similar way to what have been earlier described, the processing unit is adapted to determine the pose of the locating transducers A, AA; B, BB of each locating units 10a, 10b based on the measurement of the concerned magnetic fields and to determine the pose of the second locating transducer BB of the second locating unit 10b with respect to the second locating transducer AA of the first locating unit 10a based on the determined poses of the transducers of each locating unit and on the link L formed between said locating units. Thus, the processing unit is adapted to determine, in real-time, the pose of the surgical tool held by the end effector of the robotic arm with respect to the anatomical structures of interest, here the tibia and the talus.

### Augmented reality device

**Figure 13** illustrates a particular application of the invention, wherein the real-time electromagnetic localization system 1 is used concurrently with an augmented reality (AR) device 200. Especially, figure 13 illustrates the spine application illustrated and described with reference to figure 8a associated with said AR device 200. The part of the set-up which is identical to the one represented on figure 8a will not be described again as the description given above applies *mutatis mutandis.*

According to the illustrated example, the system further comprises two extra locating units 10'a, 10e. One of these extra locating units 10'a comprises the first locating transducer A of the first locating unit 10a and a second locating transducer AAA attached to a chest protector 201 worn by the user U of the system. The other of these extra locating units 10e comprises a first locating transducer E attached to the AR device 200 and a second locating transducer EE attached to the chest protector 201 worn by the user U of the system. Thus, the second locating transducers AAA, EE of the extra locating units 10'a, 10e are linked to each other thanks to said chest protector 201. According to this example, the link L2 between the transducers of the extra locating units is thus mechanical and supported by said chest protector 201.

Using this chain of locating units, the processing unit is adapted to determine the pose of each pair of transducers of each locating units, then the pose of each pair of linked transducers and to determine, subsequently, the poses of the not-linked transducers. Thus, the processing unit is adapted to determine, in real-time, the relative positions and orientations of the AR device 200, of the end-effector and of the anatomical structures of interest with respect to one another.

According to a non-illustrated alternative, the AR device could be equipped with at least one camera. According to this alternative, an optical tracker is attached to the first locating transducer of the first locating unit. Thus, the processing unit is adapted to determine the pose of the AR device with respect to the first locating transducer of the first locating unit and, consequently, the processing unit can determine the pose of the AR device with respect to the anatomical structure to which the second locating transducer of the second locating unit is attached. Thus, this optical tracking permits to ensure that the pose determined thanks to the chain of locating transducers is correct. As such, this optical tracking forms a redundant system with respect to the electromagnetic tracking.

Obviously, the optical tracker could be attached to any other part of the real-time electromagnetic navigation system within the scope of the invention. For instance, the optical tracker could be attached to the end-effector of the robotic arm.

## Claims

1. Real-time electromagnetic localization system (1) comprising at least two locating units (10a, 10b, 10c, 10d) and a processing unit (5), each locating unit (10a, 10b, 10c, 10d) comprising at least two locating transducers (A, AA ; B, BB, C, CC; D, AA) wherein at least one locating transducer is a transmitter adapted to emit at least one magnetic field and at least one other locating transducer is a receiver adapted to receive and measure the magnetic field emitted by the transmitter, the processing unit (5) being adapted to:
- determine a pose of a first locating transducer (A) of a first locating unit (10a) with respect to a second locating transducer (AA) of the first locating unit (10a) based on the measurement of the magnetic field emitted by the transmitter of said first locating unit (10a),
- determine a pose of a first locating transducer (B) of a second locating unit (10b) with respect to a second locating transducer (BB) of the second locating unit (10b) based on the measurement of the magnetic field emitted by the transmitter of said second locating unit (10b),
- determine a pose of the first locating transducer (A) of the first locating unit (10a) with respect to the first locating transducer (B) of the second locating unit (10b) based on a mechanical link (L) formed between the first locating transducer (A) of the first locating unit (10a) and the first locating transducer (B) of the second locating unit (10b), said mechanical link being formed such that its geometry is known or can be determined,
- determine a pose of the second locating transducer (AA) of the first locating unit (10a) with respect to the second locating transducer (BB) of the second locating unit (10b) based on
- the determined pose of the first locating transducer (A) of the first locating unit (10a) with respect to the second locating transducer (AA) of the first locating unit (10a);
- the determined pose of the first locating transducer (B) of the second locating unit (10a) with respect to the second locating transducer (BB) of the second locating unit (10b);
- the determined pose of the first locating transducer (A) of the first locating unit (10a) with respect to the first locating transducer (B) of the second locating unit (10b).

2. Real-time electromagnetic localization system (1) according to claim 1 wherein the mechanical link (L) is a rigid and straight bar.

3. Real-time electromagnetic localization system (1) according to claim 1 wherein the mechanical link (L) is a rigid and curved bar.

4. Real-time electromagnetic localization system (1) according to claim 1, wherein the mechanical link (L) is mobile in translation and/or rotation and wherein the processing unit (5) is adapted to calculate a transformation determining the translation and/or rotation of the first locating transducer (A) of the first locating unit (10a) with respect to the first locating transducer (B) of the second locating unit (10b).

5. Real-time electromagnetic localization system (1) according to claim 4, wherein the mechanical link (L) is formed as an articulated device which comprises at least one joint and at least one encoder on each joint.

6. Real-time electromagnetic localization system according to claim 4, wherein the mechanical link (L) is formed as an articulated device which comprises at least one joint driven by at least one motor controlled by the processing unit (5).

7. Real-time electromagnetic localization system according to any of the preceding claims, wherein the mechanical link (L) is adapted to be mounted on an articulated device which is adapted to be locked in a fixed position.

8. Real-time electromagnetic localization system according to any of claims 1 to 6, wherein the mechanical link (L) is adapted to be mounted on a robot which is configured to adjust the position of the first locating transducer (A) of the first locating unit (10a) and the first locating transducer (B) of the second locating unit (10b) such that the first locating transducer (A) of the first locating unit (10a) is in an optimal range with respect to the second locating transducer (AA) of the first locating unit (10a) and such that the first locating transducer (B) of the second locating unit (10b) is in an optimal range with respect to the second locating transducer (BB) of the second locating unit (10b) and such that an indicator of measurement quality of the emitted magnetic fields is optimized.

9. Real-time electromagnetic localization system (1) according to any of the preceding claims, wherein the mechanical link (L) is adapted to be non-invasively attached to a patient, and/or to a patient's support device and/or to a chest protector worn by a user of the real-time electromagnetic system (1).

10. Real-time electromagnetic localization system (1) according to any of the preceding claims, wherein the mechanical link (L) comprises one or more electrically non-conductive material(s), non-magnetic material(s), and/or material(s) with low thermal expansion coefficient.

11. Real-time electromagnetic localization system (1) according to any of the preceding claims, comprising at least a third locating unit (10c) comprising at least two locating transducers (C, CC) wherein at least one locating transducer is a transmitter adapted to emit at least one magnetic field and at least one other locating transducer is a receiver adapted to receive and measure the magnetic field emitted by the transmitter, a first locating transducer (C) of the third locating unit (10c) being mechanically linked to the first locating transducer (A) of the first locating unit (10a) and a second locating transducer (CC) of the third locating unit (10c) being mechanically linked to the first locating transceiver (B) of the second locating unit (10b), said mechanical links (L1, L2) being formed such that their respective geometries are known or can be determined, the processing unit (5) being adapted to:
- determine a pose of the first locating transducers (A, C, B) of each locating unit (10a, 10B, 10c) with respect to the second locating transducers (AA, BB, CC) of each concerned locating unit (10a, 10b, 10c) based on the measurement of the magnetic field emitted by the transmitter of said each locating unit (10a, 10b, 10c);
- determine a pose of the first locating transducer (A) of the first locating unit (10a) with respect to the first locating transducer (C) of the third locating unit (10c) based on the mechanical link (L1) formed between said transducers,
- determine a pose of the first locating transducer (B) of the second locating unit (10b) with respect to the second locating transducer (CC) of the third locating unit (10c) based on the mechanical link (L2) formed between said transducers,
- determine a pose of the second locating transducer (AA) of the first locating unit (10a) with respect to the second locating transducer (BB) of the second locating unit (10b) based on
- the determined pose of the first locating transducers (A, B, C) with respect to the second locating transducers (AA, BB, CC) for each locating unit (10a, 10b, 10c),
- the determined pose of the first locating transducer (A) of the first locating unit (10a) with respect to the first locating transducer (C) of the third locating unit (10c),
- the determined pose of the first locating transducer (B) of the second locating unit (10b) with respect to the second locating transducer (CC) of the third locating unit (10c).

12. Real-time electromagnetic localization system according to the preceding claim, comprising at least a fourth locating unit (10d) comprising at least two locating transducers (D, AA) wherein at least one locating transducer is a transmitter adapted to emit at least one magnetic field and at least one other locating transducer is a receiver adapted to receive and measure the magnetic field emitted by the transmitter, a first locating transducer (D) of the fourth locating unit (10d) being mechanically linked to the second locating transducer (CC) of the third locating unit (10c) such that the geometry of said mechanical link is known or can be determined by the processing unit (5), and a second locating transducer (AA) of the fourth locating unit (10d) being shared with the first locating unit (10a), the processing unit (5) being adapted to :
- determine a pose of the first locating transducers (B, C, D) of each of the second, third and fourth locating units (10b, 10c, 10d) with respect to the second locating transducers (BB, CC, AA) of the concerned locating units (10b, 10c, 10d),
- determine a pose of the first locating transducer (D) of the fourth locating unit (10d) with respect to the second locating transducer (CC) of the third locating unit (10c) based on the mechanical link (L3) formed between said transducers,
- determine a pose of the second locating transducer (AA) of the fourth locating unit (10d) with respect to the second locating transducer (BB) of the second locating unit (10b) based on:
• the determined pose of the first locating transducers (B, C, D) of the second, third and fourth locating units (10b, 10c, 10d) with respect to the second locating transducers (BB, CC, AA) of the concerned locating units,
• the determined pose of the first locating transducer (D) of the fourth locating unit (10d) with respect to the second locating transducer (CC)
- compare the determined poses of the second locating transducer (AA) of the first locating unit (10a) with respect to the second locating transducer (BB) of the second locating unit (10b) and determine if the difference between said poses exceeds a predefined threshold.

13. Real-time electromagnetic localization system according to any of the preceding claims, wherein a working distance between the two locating transducers of each locating unit is at least three times smaller than a distance, the smallest, measured between one of the locating transducers of the concerned locating unit and the closest disturbing material.

14. A method for determining a pose of a locating transducer implemented by a real-time electromagnetic localization system comprising
at least two locating units and at least one processing unit, each locating unit comprising at least two locating transducers, wherein
at least one locating transducer of each locating unit is a transmitter emitting at least one magnetic field, and
at least one other locating transducer of each locating unit is a receiver receiving and measuring the magnetic field emitted by the transmitter,
a first locating transducer of a first locating unit being mechanically linked to a first locating transducer of a second locating unit, such that a pose of the first locating transducer of the first locating unit with respect to the first locating transducer of the second locating unit is known or can be determined; and
a processing unit;
said method comprising determining, by the processing unit,
- a pose of a first locating transducer (A) of a first locating unit (10a) with respect to a second locating transducer (AA) of the first locating unit (10a), based on the measurement of the magnetic field emitted by the transmitter of said first locating unit (10a),
- a pose of a first locating transducer (B) of a second locating unit (10b) with respect to a second locating transducer (BB) of the second locating unit (10b) based on the measurement of the magnetic field emitted by the transmitter of said second locating unit (10b),
- a pose of the first locating transducer (A) of the first locating unit (10a) with respect to the first locating transducer (B) of the second locating unit (10b) based on the mechanical link (L) formed between the first locating transducer (A) of the first locating unit (10a) and the first locating transducer (B) of the second locating unit (10b),
- a pose of the second locating transducer (AA) of the first locating unit (10a) with respect to the second locating transducer (BB) of the second locating unit (10b) based on
∘ the determined pose of the first locating transducer (A) of the first locating unit (10a) with respect to the second locating transducer (AA) of the first locating unit (10a)
∘ the determined pose of the first locating transducer (B) of the second locating unit (10b) with respect to the second locating transducer (BB) of the second locating unit (10b), and on
∘ the determined pose of the first locating transducer (A) of the first locating unit (10a) with respect to the first locating transducer (B) of the second locating unit (10b).

15. The method for determining the pose of a locating transducer according to the preceding claim, wherein the first locating transducer (A) of the first locating unit (10a) is mechanically linked to the first locating transducer (B) of the second locating unit (10b); wherein said method comprises an initial step of self-calibrating for determining an initial pose of the first locating transducer (A) of the first locating unit (10a) with respect to the first locating transducer (B) of the second locating unit (10b) by using a calibration transducer (13) disposed proximately to the mechanical link between said first locating transducers (A, B), wherein
if one of the first locating transducers is a transmitter, the calibration transducer receives and measures a magnetic field emitted by the transmitter;
if one of the first locating transducer is a receiver, said receiver receives and measures a magnetic field transmitted by the calibration transducer; and wherein the processing unit determines from these measurements a pose of the first locating transducer of the first locating unit with respect to the first locating transducer of the second locating unit to determine a geometry of the mechanical link between said first and locating transducers.

16. A computer program product comprising program code instructions for the execution of the steps of the method according to one of the claims 14 or 15 when said program is executed by a processing unit of a real-time electromagnetic localization system according to one of the claims 1 to 13.
